# NEW EUROPEAN PATENT SPECIFICATION

(11) **EP 0 656 203 B3**
(45) Date of publication of this specification: **26.01.2011**
(45) Mention of the grant of the patent: 06.05.1999
(21) Application number: 94918886.6
(22) Date of filing: 21.06.1994
(51) Int. Cl.: A61K 9/12

(54) **INJECTABLE MICROFOAM CONTAINING A SCLEROSING AGENT**
Injizierbarer Mikroschaum, der ein Verödungsmittel enthält
MICROMOUSSE INJECTABLE CONTENANT UN AGENT SCLEROSANT

(30) Priority: 23.06.1993 ES 9300141
(43) Date of publication of application: 07.06.1995
(73) Proprietor: BTG INTERNATIONAL LIMITED, London EC4M 7RD (GB)
(72) Inventor: Cabrera Garrido, Juan, 18005 Granada (ES); Cabrera Garcia-Olmedo, Juan, 18005 Granada (ES)
(74) Representative: Dolan, Anthony Patrick
(86) International application number: PCT/ES1994/000064
(87) International publication number: WO 1995/000120

(56) References cited:
- EP-A- 0 077 752
- WO-A-92/05806

## Description

### State of the art

Sclerosis of varicose veins is based on the injection into the veins of liquid substances which, by causing a localized inflammatory reaction, favours the elimination of these abnormal veins.

When a sclerosing liquid is injected, it is mixed with the blood contained in the vein and is diluted in an unknown proportion. The results are uncertain (owing to over- or under-dosage) and are limited to short varicose segments.

As the size of the varicose veins to be injected decreases, this dilution is less and the results obtained are more predictable. Nowadays sclerosis is a technique selected in cases of small and medium varicose veins, those with diameters equal to or greater than 7 mm being treated by surgery.

At present, sclerosis and surgery complement one another but sclerosis treatment continues not to be applicable to large varicose veins.

In these large varicose veins, if a sclerosing substance is injected, its concentration in the vein, its homogeneous distribution in the blood, and the time for which it is in contact with the internal walls of the vessel treated are not known.

In 1946, Orbach injected a few cubic centimetres of air into small varicose veins and confirmed a displacement of the blood inside the vessel which was occupied by the injected air. The sclerosing solution introduced immediately afterwards was more effective than if it had been injected into the blood.

In thick varicose veins, when air is injected the phenomenon described of the displacement of the blood by the injected air does not occur but the air forms a bubble inside the vein which makes the method ineffective in these vessels.

The same author had the idea, a few years later, of injecting foam obtained by agitation of a container containing sodium tetradecyl sulphate, which is an anionic sclerosing detergent with a good foaming capability.

The method was of little use owing to the large size of the bubbles formed and was dangerous owing to the side effects of atmospheric nitrogen which is only slightly soluble in blood.

Both methods had limited practical repercussion being used only in small varicose veins.

### Description of the invention

This invention relates to the preparation of An injectable micro-foam for therapeutic uses, prepared or for preparation as required, **characterized in that** the micro-foam is produced with any sclerosing substance..

According to the present invention, it has been discovered that if a micro-foam of a pharmacologically inert, sterile, physiological serum is injected in a horizontal position, the micro-foam causes displacement of the blood contained in the vessel, including the most expanded varicose veins, on account of the low pressure of the blood contained therein in the horizontal.

The elevation of the member injected reduces the venous pressure even more facilitating the filling of the vein exclusively with micro-foam, which remains in the vessel as long as the patient does not get up from the operating table.

If the micro-foam produced with the physiological serum is replaced by micro-foam produced with a sclerosing substance and injected into the vein, it displaces the blood contained in the vein and ensures that the sclerosing agent contacts the endothelium of the vessel in a known concentration and for a controllable time, achieving sclerosis of the entire segment occupied.

The advantages of this method allow:
1. the concentration of the sclerosing agent in the vessel to be known, since the micro-foam displaces the blood and is not diluted therein like a liquid;
2. homogeneous distribution of the sclerosis product therein to be insured;
3. the time for which it is kept in contact with the internal walls of the vein to be controlled;
none of which factors is known precisely or is controllable with the use of liquid sclerosing agents.

The present invention can be implemented by the preparation of a micro-foam with any sclerosing substance such as: polidocanol, sodium tetradecyl sulphate, hypertonic glucose or gluco-saline solutions, chromic glycerol, ethanolamine oleate, sodium morrhuato, or iodic solutions.

Further therapeutic uses of the injectable micro-foam of the invention are given in Claims 9 to 12.

Once the sclerosing micro-foam has been produced by any of the existing methods, of which two are described below, it is introduced into any sterile container which can serve for subsequent injection into the veins to be treated and which permits stability of the foam in a form which can be extracted by means of a syringe or any other instrument which facilitates its injection into the vessels to be treated.

### Example 1:

The sclerosing micro-foam was produced by mixing in a sterile, hermetic container connected, if desired, to a pressure bottle of oxygen, or a mixture of oxygen and carbon dioxide or other physiological gases; mechanical beating was carried out by means of a micro-motor which rotated a brush immersed in the sclerosing solution to be foamed.

The micro-foam was produced by beating at between 8,000 and 15,000 rpm for a period of between 60 and 120 seconds.

It was introduced into any container which could later serve for its storage and its subsequent injection into the vessels to be sclerosed.

If the sclerosing substance does not have a foaming capability, polysorbate 20, polysorbate 80, polygelina or any other substance with a foaming capability accepted as inert for intravenous use is added.

### Example 2:

The sclerosing substance was introduced into a hermetic, pressurized and sterile container and the micro-foam was produced by stirring the solution with discharge from the container for subsequent use.

## Claims

1. An injectable micro-foam for therapeutic uses, prepared or for preparation as required, **characterized in that** the micro-foam is produced with any sclerosing substance by foaming with oxygen or a mixture of oxygen and carbon dioxide gas.

2. An injectable micro-foam for therapeutic uses according to claim 1, **characterized in that** the sclerosing substance is polidocanol.

3. An injectable micro-foam for therapeutic uses according to claim 1, **characterized in that** the sclerosing substance is sodium tetradecyl sulphate

4. An injectable micro-foam for therapeutic uses according to claim 1, **characterized in that** the sclerosing substance is a hypertonic glucose or gluco-saline solution.

5. An injectable micro-foam for therapeutic uses according to claim 1, **characterized in that** the substance used is chromic glycerol.

6. An injectable micro-foam for therapeutic uses according to claim 1, **characterized in that** the substance used is ethanolamine oleate.

7. An injectable micro-foam for therapeutic uses according to claim 1, **characterized in that** the substance used is sodium morrhuate.

8. An injectable micro-foam for therapeutic uses according to claim 1, **characterized in that** the substance used is any iodic solution.

9. An injectable micro-foam for therapeutic uses according to the preceding claims for use in phlebology.

10. An injectable micro-foam for therapeutic uses according to claims 1 to 8, for use in the treatment of oesophageal varices.

11. An injectable micro-foam for therapeutic uses according to claims 1 to 8, for use in proctology.

12. An injectable micro-foam for therapeutic uses according to claims 1 to 8, for use in angiology.

13. A method for the preparation of an injectable micro-foam for use in therapy **characterised in that** it comprises producing a micro-foam with a sclerosing substance and oxygen or a mixture of oxygen and carbon dioxide.

14. A method as claimed in claim 13 **characterised in that** the sclerosing substance is a polidocanol, sodium tetradecyl sulphate, hypertonic glucose or gluco-saline solution, chromic glycerol, ethanolamine oleate, sodium morrhuato or iodic solution.

15. A micro-foam for use in therapy **characterised in that** it is obtainable by beating a sclerosing solution with a micromotor rotated brush at 8,000 to 15,000 r.p.m. for 60 to 120 seconds wherein the sclerosing substance is a polidocanol, sodium tetradecyl sulphate, hypertonic glucose or gluco-saline solution, chromic glycerol, sodium morrhuato or iodic solution.

16. A microfoam as claimed in Claim 15 made with oxygen or a mixture of oxygen and carbon dioxide.

## Patentansprüche

1. Injizierbarer Mikroschaum für therapeutische Anwendungen, hergestellt oder zur Herstellung nach Bedarf, **dadurch gekennzeichnet, dass** der Mikroschaum mit irgendeiner sklerosierenden Substanz durch Aufschäumen mit Sauerstoff oder einer Mischung von Sauerstoff und Kohlendioxidgas erzeugt wird.

2. Injizierbarer Mikroschaum für therapeutische Anwendungen nach Anspruch 1, **dadurch gekennzeichnet, dass** die sklerosierende Substanz Polidocanol ist.

3. Injizierbarer Mikroschaum für therapeutische Anwendungen nach Anspruch 1, **dadurch gekennzeichnet, dass** die sklerosierende Substant Natriumtetradecylsulfat ist.

4. Injizierbarer Mikroschaum für therapeutische Anwendungen nach Anspruch 1, **dadurch gekennzeichnet, dass** die sklerosierende Substanz eine hypertonische Glucose- oder Glucose/Kochsalz-Lösung ist.

5. Injizierbarer Mikroschaum für therapeutische Anwendungen nach Anspruch 1, **dadurch gekennzeichnet, dass** die verwendete Substanz Chromglycerin ist.

6. Injizierbarer Mikroschaum für therapeutische Anwendungen nach Anspruch 1, **dadurch gekennzeichnet, dass** die verwendete Substanz Ethanolaminoleat ist.

7. Injizierbarer Mikroschaum für therapeutische Anwendungen nach Anspruch 1, **dadurch gekennzeichnet, dass** die verwendete Substanz Natriummorrhuat ist.

8. Injizierbarer Mikroschaum für therapeutische Anwendungen nach Anspruch 1, **dadurch gekennzeichnet, dass** die verwendete Substanz irgendeine Jodlösung ist.

9. Injizierbarer Mikroschaum für therapeutische Anwendungen nach den vorangehenden Ansprüchen zur Verwendung in der Phlebologie.

10. Injizierbarer Mikroschaum für therapeutische Anwendungen nach Ansprüchen 1 bis 8, zur Verwendung bei der Behandlung von Ösophagusvarizen.

11. Injizierbarer Mikroschaum für therapeutische Anwendungen nach Ansprüchen 1 bis 8, zur Verwendung in der Proktologie.

12. Injizierbarer Mikroschaum für therapeutische Anwendungen nach Ansprüchen 1 bis 8, zur Verwendung in der Angiologie.

13. Verfahren zur Herstellung eines injizierbaren Mikroschaums zur Verwendung bei der Therapie, **dadurch gekennzeichnet, dass** es die Herstellung eines Mikroschaums mit einer sklerosierenden Substanz und Sauerstoff oder einer Mischung von Sauerstoff und Kohlendioxid umfasst.

14. Verfahren nach Anspruch 13, **dadurch gekennzeichnet, dass** die sklerosierende Substanz ein Polydocanol, Natriumtetradecylsulfat, hypertonische Glucose- oder Glucose/Kochsalz-Lösung, Chromglycerin, Ethanolaminoleat, Natriummorrhuat oder Jodlösung ist.

15. Mikroschaum zur Verwendung bei der Therapie, **dadurch gekennzeichnet, dass** er erhältlich ist durch Aufschlagen einer sklerosierenden Lösung mit einer durch einen Mikromotor angetriebenen rotierenden Bürste bei 8000 bis 15000 UpM 60 bis 120 Sekunden, wobei die sklerosierende Substanz ein Polydocanol, Natriumtetradecylsulfat, hypertonische Glucose- oder Glucose/Kochsalz-Lösung, Chromglyzerin, Natriummorrhuat oder Jodlösung ist.

16. Mikroschaum nach Anspruch 15, hergestellt mit Sauerstoff oder einer Mischung von Sauerstoff und Kohlendioxid.

## Revendications

1. Micromousse injectable pour usages thérapeutiques, préparée ou destinée à une préparation selon les besoins, **caractérisée en ce que** la micromousse est produite avec une substance sclérosante quelconque par formation de mousse avec de l'oxygène ou un mélange d'oxygène et de dioxyde de carbone gazeux.

2. Micromousse injectable pour usages thérapeutiques selon la revendication 1, **caractérisée en ce que** la substance sclérosante est le polidocanol.

3. Micromousse injectable pour usages thérapeutiques selon la revendication 1, **caractérisée en ce que** la substance sclérosante est le tétradécyl-sulfate de sodium.

4. Micromousse injectable pour usages thérapeutiques selon la revendication 1, **caractérisée en ce que** la substance sclérosante est une solution hypertonique de glucose ou glucosaline.

5. Micromousse injectable pour usages thérapeutiques selon la revendication 1, **caractérisée en ce que** la substance utilisée est du glycérol chromique.

6. Micromousse injectable pour usages thérapeutiques selon la revendication 1, **caractérisée en ce que** la substance utilisée est l'oléate d'éthanolamine.

7. Micromousse injectable pour usages thérapeutiques selon la revendication 1, **caractérisée en ce que** la substance utilisée est le morrhuate de sodium.

8. Micromousse injectable pour usages thérapeutiques selon la revendication 1, **caractérisée en ce que** la substance utilisée est une solution iodique quelconque.

9. Micromousse injectable pour usages thérapeutiques selon les revendications précédentes, destinée à être utilisée en phlébologie.

10. Micromousse injectable pour usages thérapeutiques selon les revendications 1 à 8, destinée à être utilisée dans le traitement des varices oesophagiennes.

11. Micromousse injectable pour usages thérapeutiques selon les revendications 1 à 8, destinée à être utilisée en proctologie.

12. Micromousse injectable pour usages thérapeutiques selon les revendications 1 à 8, destinée à être utilisée en angiologie.

13. Méthode de préparation d'une micromousse injectable destinée à être utilisée en thérapie, **caractérisée en ce qu'**elle comprend la production d'une micromousse avec une substance sclérosante et de l'oxygène ou un mélange d'oxygène et de dioxyde de carbone.

14. Méthode telle que revendiquée dans la revendication 13, **caractérisée en ce que** la substance sclérosante est le polidocanol, le tétradécyl-sulfate de sodium, une solution hypertonique de glucose ou glucosaline, du glycérol chromique, l'oléate d'éthanolamine, le morrhuate de sodium ou une solution iodique.

15. Micromousse pour son usage en thérapie, **caractérisée en ce qu'**elle peut être obtenue en battant une solution sclérosante avec une brosse d'un micromoteur rotatif entre 8 000 et 15 000 t/min pendant 60 à 120 secondes, dans laquelle la substance sclérosante est le polidocanol, le tétradécyl-sulfate de sodium, une solution hypertonique de glucose ou glucosaline, du glycérol chromique, le morrhuate de sodium ou une solution iodique.

16. Micromousse selon la revendication 15, faite avec de l'oxygène ou un mélange d'oxygène et de dioxyde de carbone.
